(19) **Europäisches Patentamt European Patent Office Office européen des brevets**

(11) **EP 4 781 972 A1**

(12) **EUROPEAN PATENT APPLICATION**
published in accordance with Art. 153(4) EPC

(43) Date of publication:
**29.07.2026 Bulletin 2026/31**

(21) Application number: **24868524.0**

(22) Date of filing: **02.09.2024**

(51) International Patent Classification (IPC):
*A61K 8/35* (2006.01)　　*A61K 8/34* (2006.01)
*A61K 8/37* (2006.01)　　*A61Q 19/00* (2006.01)
*A61K 47/10* (2017.01)　　*A61K 47/14* (2017.01)
*A61Q 17/00* (2006.01)

(52) Cooperative Patent Classification (CPC):
**A61K 8/34; A61K 8/35; A61K 8/37; A61K 8/42;
A61K 47/10; A61K 47/14; A61K 47/18;
A61P 17/00; A61Q 17/00; A61Q 19/00**

(86) International application number:
**PCT/KR2024/013163**

(87) International publication number:
**WO 2025/063545 (27.03.2025 Gazette 2025/13)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **19.09.2023 KR 20230124816
15.03.2024 KR 20240036722**

(71) Applicant: **Activon Co., Ltd.
Cheongju-si, Chungcheongbuk-do 28104 (KR)**

(72) Inventors:
• **KIM, Myo Deok
Cheongju-si, Chungcheongbuk-do 28104 (KR)**
• **KIM, Dae Gun
Cheongju-si, Chungcheongbuk-do 28104 (KR)**
• **JEON, Na Young
Cheongju-si, Chungcheongbuk-do 28104 (KR)**
• **PARK, Byung Gyu
Cheongju-si, Chungcheongbuk-do 28104 (KR)**
• **CHO, Youn Ki
Cheongju-si, Chungcheongbuk-do 28104 (KR)**

(74) Representative: **Casci, Tamara et al
Barzanò & Zanardo S.p.A.
Via Borgonuovo, 10
20121 Milano (IT)**

(54) **PRESERVATIVE FOR EXTERNAL APPLICATION TO SKIN COMPRISING HINOKITIOL AND COSMETIC COMPOSITION COMPRISING SAME**

(57) The present disclosure provides a preservative for external skin preparation and a use thereof, the preservative including: hinokitiol; and at least one solvent selected from the group consisting of alkanetriol having 3 to 6 carbon atoms, dipropylene glycol, glyceryl caprylate, ethylhexylglycerin, and phenoxyethanol. According to the preservative for external skin preparation and the like according to one aspect, a liquid state can be stably maintained at room temperature under a refrigerated condition, and an excellent antimicrobial effect can be exhibited even after the formulation.

## Description

### Technical Field

The present application claims Korean Patent Application No. 10-2023-0124816 filed on September 19, 2023 and Korean Patent Application No. 10-2024-0036722 filed on March 15, 2024 as a priority, and the disclosures of the patent application are incorporated herein by reference in the present specification.

The present application relates to a preservative for external skin preparation including hinokitiol, and a cosmetic composition including the same.

### Background Art

Since cosmetic products cannot avoid contamination by microorganisms during the manufacturing process or use process, a preservative capable of improving the preservative efficacy of the product is essentially required in manufacturing cosmetic products. Examples of the preservative include a parabens preservative, imidazolidinylurea, and phenoxyethanol. However, although the above-described preservative has the advantage of being very excellent in preservative efficacy due to its high antimicrobial power, it is also widely known for its disadvantages of causing toxicity, skin irritation, allergies, and the like. Therefore, there is a need to develop a safe preservative that does not cause skin irritation and has high preservative efficacy.

As part of such research, the development of preservatives using diols having known antimicrobial power has continued. However, since alkanediol-based ingredients are known to cause toxicity, skin irritation, or allergic reactions, it is necessary to minimize their concentration. In such cases, there is a drawback in that the preservative effect is inevitably compromised.

Therefore, there is a need to develop a preservative for external skin preparation that is stable while having excellent preservative efficacy without causing skin irritation.

Under this background, the present inventors have made extensive efforts to develop a preservative for external skin preparation having excellent formulation stability, and as a result, have confirmed that a preservative for external skin preparation having excellent formulation stability and excellent antiseptic power can be prepared through a combination of hinokitiol and various compounds, thereby completing the present application.

### Disclosure of Invention

### Technical Problem

An aspect provides a preservative for external skin preparation, the preservative including hinokitiol; and at least one solvent selected from the group consisting of alkanediol having 3 to 10 carbon atoms, alkanetriol having 3 to 6 carbon atoms, dipropylene glycol, glyceryl caprylate, ethylhexylglycerin, and phenoxyethanol.

Another aspect provides a cosmetic composition including the preservative for external skin preparation.

Still another aspect provides a pharmaceutical composition for external skin preparation, the pharmaceutical composition including the preservative for external skin preparation.

Still another aspect provides a method of preparing a cosmetic composition, the method including adding the preservative for external skin preparation.

Still another aspect provides a method of performing antimicrobial, preservation, or antiseptic treatment on a cosmetic composition, the method including introducing a preservative for external skin preparation.

Another aspect provides use of a composition for external skin preparation or a cosmetic composition for antimicrobial, preservation, or antiseptic treatment, the composition including hinokitiol; and at least one solvent selected from the group consisting of alkanediol having 3 to 10 carbon atoms, alkanetriol having 3 to 6 carbon atoms, dipropylene glycol, glyceryl caprylate, ethylhexylglycerin, and phenoxyethanol.

Other objects and advantages of the present disclosure will become more apparent from the following detailed description together with the appended claims and drawings. Descriptions of matters not explicitly detailed in the present specification are omitted, as they can be sufficiently understood and derived by a person skilled in the art to which the present application pertains or in similar technical fields.

### Solution to Problem

Each description and embodiment disclosed in this application may also apply to each other description and embodiment. That is, all combinations of various elements disclosed in the present application fall within the scope of the present application. In addition, it may not be considered that the scope of the present application is limited by the detailed description described below.

An aspect provides a preservative for external skin preparation including: hinokitiol; and at least one solvent selected from the group consisting of alkanediol having 3 to 10 carbon atoms, alkanetriol having 3 to 6 carbon atoms, dipropylene glycol, glyceryl caprylate, ethylhexylglycerin, and phenoxyethanol.

The term "external skin preparation" as used herein is a concept encompassing compositions in general applied to the skin, and includes, for example: various cosmetics such as basic cosmetics, makeup cosmetics, and hair care cosmetics; and pharmaceutical compositions for topical administration, including pharmaceuticals and quasi-drugs in the form of ointments, creams, and lotions, and the like.

The term "preservative for external skin preparation" as used herein may be interchangeably used with "an antimicrobial agent," "an antiseptic agent," "an antimicrobial preservative," "an antiseptic preservative," "an antimicrobial composition," "an antiseptic composition," "an antimicrobial or antiseptic composition," "an antimicrobial cosmetic composition," "an antiseptic cosmetic composition," "an antimicrobial or antiseptic cosmetic composition," and the like.

The term "antimicrobial" as used herein refers to resistance to all contaminated microorganisms such as bacteria, molds, yeast, etc., the term "antimicrobial power" or "antimicrobial effect" refers to a protective power or a protective effect against these contaminated microorganisms, the term "antiseptic power" or "antiseptic effect" refers to a protective power or a protective effect against decomposition caused by contamination by microorganisms, and is a concept including an antimicrobial power or an antimicrobial effect.

The term "preservative" as used herein refers to a substance that has antiseptic power or antioxidant power to prevent deterioration of a product for a long period of time and maintain its original state, and the term "preservative efficacy" refers to a protective power that can prevent deterioration of a product for a long period of time and maintain its original state.

Conventional preservatives, despite effective antimicrobial activity, have difficulties in mass production and processing due to instability caused by storage conditions, for example, deterioration such as solidification. Therefore, there is a need to develop a preservative with effective antimicrobial activity and high stability at room temperature or in refrigerated conditions.

The inventors of the present applications have attempted to develop a naturally derived preservative for external skin preparation, which can maintain a liquid state, particularly, a liquid state at room temperature and in a refrigerated condition, in terms of processing convenience and management, and can secure excellent antimicrobial power and preservative efficacy compared to conventional preservatives.

In an embodiment, the preservative may include hinokitiol and the solvent in a weight ratio of about 1:19 to about 1:1000. For example, the weight ratio may be 1:19 to 1:1000, 1:19 to 1:500, 1:19 to 1:300, 1:19 to 1:200, 1:19 to 1:100, 1:19 to 1:99, 1:19 to 1:89, 1:20 to 1:1000, 1:20 to 1:500, 1:20 to 1:300, 1:20 to 1:200, 1:20 to 1:100, 1:20 to 1:99, or 1:20 to 1:89. Specifically, the weight ratio may be 1:19 to 1:99. When an amount of the preservative is out of these ranges, the antimicrobial or protective effect of the preservative may be reduced, and deterioration of stability, such as solidification, precipitation, occurrence of precipitation, decrease in solubility, decrease in emulsification or solubilization, may be caused.

According to an experimental example, a preservative including hinokitiol and a solvent in a weight ratio within these ranges maintained a liquid state at room temperature and likewise showed excellent antimicrobial power (see Experimental Examples 2, 3, 4, and 5).

The preservative for external skin preparation may include, based on the total weight thereof, 0.1 to 5 wt% of hinokitiol. For example, hinokitiol may be included in an amount of 0.1 to 5 wt%, 0.1 to 3 wt%, 0.1 to 2 wt%, 0.1 to 1 wt%, 0.5 to 5 wt%, 0.5 to 3 wt%, 0.5 to 2 wt%, 0.5 to 1 wt%, 1 to 5 wt%, 1 to 3 wt%, or 1 to 2 wt%. When an amount of the preservative is out of these ranges, the antimicrobial or preservative effect of the preservative may be reduced, and solidification, precipitation may occur, solubility may be reduced, emulsifying power or solubilizing power may be reduced.

The preservative for external skin preparation may include, based on the total weight thereof, 85 to 99.9 wt% of alkanediol having 3 to 10 carbon atoms, alkanetriol having 3 to 6 carbon atoms, dipropylene glycol, glyceryl caprylate, ethylhexylglycerin, or phenoxyethanol. For example, alkanediol having 3 to 10 carbon atoms, alkanetriol having 3 to 6 carbon atoms, dipropylene glycol, glyceryl caprylate, ethylhexylglycerin, or phenoxyethanol may be included in an amount of 85 to 99.9 wt%, 85 to 99 wt%, 85 to 97 wt%, 85 to 95 wt%, or 85 to 90 wt%. Outside these ranges, the antimicrobial or preservative effect of the preservative may be reduced, and solidification and precipitation may occur, solubility may be reduced, emulsifying power or solubilizing power may be reduced.

The preservative for external skin preparation may include, based on the total weight thereof, 0.1 to 5 wt%, 0.1 to 3 wt%, 0.1 to 2 wt%, 0.1 to 1 wt%, 0.5 to 5 wt%, 0.5 to 3 wt%, 0.5 to 2 wt%, 0.5 to 1 wt%, 1 to 5 wt%, 1 to 3 wt%, or 1 to 2 wt% of hinokitiol; and 85 to 99.9 wt%, 85 to 99 wt%, 85 to 97 wt%, 85 to 95 wt%, or 85 to 90 wt% of at least one compound selected from the group consisting of alkanediol having 3 to 10 carbon atoms, alkanetriol having 3 to 6 carbon atoms, dipropylene glycol, glyceryl caprylate, ethylhexylglycerin, or phenoxyethanol.

The preservative for external skin preparation may further include caprylhydroxamic acid or raspberry ketone.

In an embodiment, the preservative for external skin preparation may include hinokitiol, at least one solvent selected from the group consisting of alkanediol having 3 to 10 carbon atoms, alkanetriol having 3 to 6 carbon atoms, dipropylene glycol, glyceryl caprylate, ethylhexylglycerin and phenoxyethanol, and either caprylhydroxamic acid or raspberry ketone, in a weight ratio of hinokitiol to the at least one solvent and either caprylhydroxamic acid or raspberry ketone, ranging from 1:19 to 1:1000. For example, the weight ratio may be 1:19 to 1:1000, 1:19 to 1:500, 1:19 to 1:300, 1:19 to 1:200, 1:19 to 1:100, 1:19 to 1:99, 1:19 to 1:89, 1:20 to 1:1000, 1:20 to 1:500, 1:20 to 1:300, 1:20 to 1:200, 1:20 to 1:100, 1:20 to 1:99, or 1:20 to 1:89. When an amount of the preservative is out of these ranges, the antimicrobial or antiseptic effect of the preservative may be reduced, and deterioration of stability, such as solidification, precipitation, occurrence of precipitation, decrease in solubility, decrease in emulsification or solubilization, may be caused.

In an embodiment, the preservative for external skin preparation may include the weight ratio of hinokitiol: at least one solvent selected from the group consisting of alkanediol having 3 to 10 carbon atoms, alkanetriol having 3 to 6 carbon atoms, dipropylene glycol, glyceryl caprylate, ethylhexylglycerin, and phenoxyethanol: caprylhydroxamic acid or raspberry ketone of 1 to 5 : 80 to 94 : 1 to 15. For example, the weight ratio may be 1 to 5 : 80 to 94 : 1 to 15, 1 to 5 : 80 to 93 : 2 to 15, 1 to 5 : 80 to 92 : 3 to 15, 1 to 5 : 80 to 91 : 4 to 15, 1 to 5 : 80 to 90 : 5 to 15, 1 to 5 : 80 to 89 : 6 to 15, 1 to 5 : 80 to 88 : 7 to 15, 1 to 5 : 80 to 87 : 8 to 15, 1 to 5 : 80 to 86 : 9 to 15, 1 to 5 : 80 to 85 : 10 to 15, 5 : 85 : 10 or 5 : 80 : 15. Specifically, the weight ratio may be 5 : 80 to 85 : 10 to 15.

The preservative for external skin preparation may include, based on the total weight thereof, 0.1 to 5 wt% of hinokitiol. For example, hinokitiol may be included in an amount of 0.1 to 5 wt%, 0.1 to 3 wt%, 0.1 to 2 wt%, 0.1 to 1 wt%, 0.5 to 5 wt%, 0.5 to 3 wt%, 0.5 to 2 wt%, 0.5 to 1 wt%, 1 to 5 wt%, 1 to 3 wt%, or 1 to 2 wt%. Outside these ranges, the antimicrobial or preservative effect of the preservative may be reduced, and solidification and precipitation may occur, solubility may be reduced, emulsifying power or solubilizing power may be reduced.

The preservative for external skin preparation may include, based on the total weight thereof, 75 to 94.9 wt% of alkanediol having 3 to 10 carbon atoms, alkanetriol having 3 to 6 carbon atoms, dipropylene glycol, glyceryl caprylate, ethylhexylglycerin, or phenoxyethanol. For example, alkanediol having 3 to 10 carbon atoms, alkanetriol having 3 to 6 carbon atoms, dipropylene glycol, glyceryl caprylate, ethylhexylglycerin, or phenoxyethanol may be included in an amount of 75 to 94.9 wt%, 80 to 94 wt%, 80 to 92 wt%, 80 to 90 wt%, or 80 to 85 wt%. Outside these ranges, the antimicrobial or preservative effect of the preservative may be reduced, and solidification and precipitation may occur, solubility may be reduced, emulsifying power or solubilizing power may be reduced.

The preservative for external skin preparation may include, based on the total weight thereof, 1 to 20 wt% of caprylhydroxamic acid or raspberry ketone. For example, caprylhydroxamic acid or raspberry ketone may be included in an amount of 1 to 20 wt%, 1 to 15 wt%, 1 to 10 wt%, 1 to 5 wt%, 1 to 3 wt%, 3 to 20 wt%, 3 to 15 wt%, 3 to 10 wt%, 3 to 5 wt%, 5 to 20 wt%, 5 to 15 wt%, 5 to 10 wt%, 10 to 20 wt%, or 10 to 15 wt%. Outside these ranges, the antimicrobial or preservative effect of the preservative may be reduced, and solidification and precipitation may occur, solubility may be reduced, emulsifying power or solubilizing power may be reduced.

According to an experimental example, a preservative including caprylhydroxamic acid or raspberry ketone in an amount within the above range showed excellent antiseptic power (see Experimental Example 8).

In an embodiment, in the preservative for external skin preparation, a weight ratio of hinokitiol to at least one solvent selected from the group consisting of alkanediol having 3 to 10 carbon atoms, alkanetriol having 3 to 6 carbon atoms, dipropylene glycol, glyceryl caprylate, ethylhexylglycerin, and phenoxyethanol, and either caprylhydroxamic acid or raspberry ketone is 1:19 to 1:1000, 1:19 to 1:500, 1:19 to 1:300, 1:19 to 1:200, 1:19 to 1:100, 1:19 to 1:99, 1:19 to 1:89, 1:20 to 1:1000, 1:20 to 1:500, 1:20 to 1:300, 1:20 to 1:200, 1:20 to 1:100, 1:20 to 1:99, or 1:20 or 1:89, and caprylhydroxamic acid or raspberry ketone is included in the amount of 1 wt% to 20 wt%, 1 wt% to 15 wt%, 1 wt% to 10 wt%, 1 wt% to 5 wt%, 1 wt% to 3 wt%, 3 wt% to 20 wt%, 3 wt% to 15 wt%, 3 wt% to 10 wt%, 3 wt% to 5 wt%, 5 wt% to 20 wt%, 5 wt% to 15 wt%, 5 wt% to 10 wt%, 10 wt% to 20 wt%, or 10 wt% to 15 wt%. When the preservative satisfies these weight ratios of hinokitiol to at least one solvent selected from the group consisting of alkanediol having 3 to 10 carbon atoms, alkanetriol having 3 to 6 carbon atoms, dipropylene glycol, glyceryl caprylate, ethylhexylglycerin, and phenoxyethanol, and either caprylhydroxamic acid or raspberry ketone; and the amount range of the caprylhydroxamic acid or raspberry ketone, antimicrobial power and stability may be remarkably improved.

The preservative for external skin preparation may include: hinokitiol in an amount of 0.1 to 5 wt%, 0.1 to 3 wt%, 0.1 to 2 wt%, 0.1 to 1 wt%, 0.5 to 5 wt%, 0.5 to 3 wt%, 0.5 to 2 wt%, 0.5 to 1 wt%, 1 to 5 wt%, 1 to 3 wt%, or 1 to 2 wt%; at least one compound selected from the group consisting of alkanediol having 3 to 10 carbon atoms, alkanetriol having 3 to 6 carbon atoms, dipropylene glycol, glyceryl caprylate, ethylhexylglycerin, or phenoxyethanol may be included in an amount of 75 to 94.9 wt%, 80 to 94 wt%, 80 to 92 wt%, 80 to 90 wt%, or 80 to 85 wt%; and caprylhydroxamic acid or raspberry ketone in an amount of 1 to 20 wt%, 1 to 15 wt%, 1 to 10 wt%, 1 to 5 wt%, 1 to 3 wt%, 3 to 20 wt%, 3 to 15 wt%, 3 to 10 wt%, 3 to 5 wt%, 5 to 20 wt%, 5 to 15 wt%, 5 to 10 wt%, 10 to 20 wt%, or 10 to 15 wt%, based on the total weight of the preservative for external skin preparation.

In an embodiment, alkanediol having 3 to 10 carbon atoms may be propanediol, methylpropanediol, butanediol, pentanediol, hexanediol, heptanediol, octanediol, or decanediol, and more specifically, may be at least one selected from the group consisting of 1,3-propanediol, methylpropanediol, 2,3-butanediol, 1,3-butanediol, 1,2-pentanediol, 1,5-pentanediol, 1,2-hexanediol, 1,2-heptanediol, 1,2-octanediol, and 1,2-decanediol.

In an embodiment, the alkanetriol having 3 to 6 carbon atoms may be glycerin, 1,2,6-hexanetriol, or a combination thereof.

In another embodiment, the solvent may be at least one selected from the group consisting of 1,3-propanediol, methylpropanediol, 2,3-butanediol, 1,3-butanediol, 1,2-pentanediol, 1,5-pentanediol, 1,2-hexanediol, 1,2-heptanediol, 1,2-octanediol, 1,2-decanediol, glycerin, 1,2,6-hexanetriol, dipropylene glycol, glyceryl caprylate, ethylhexylglycerin, and phenoxyethanol. More specifically, the solvent may be 1,2-pentanediol, 1,5-pentanediol, 1,2-octanediol, glyceryl caprylate, or a combination thereof.

According to one experimental example, a preservative including: hinokitiol; and alkanediol having 3 to 10 carbon atoms, alkanetriol having 3 to 6 carbon atoms, dipropylene glycol, glyceryl caprylate, ethylhexylglycerin, or phenoxyethanol maintained a liquid state at room temperature and showed excellent antimicrobial power against bacteria, yeasts, and molds.

In addition, according to one experimental example, it was confirmed that 1,2-pentanediol, 1,5-pentanediol, 1,2-octanediol, or glyceryl caprylate exhibited a synergistic antimicrobial effect with hinokitiol against strains.

The compound may be obtained by a method known to those skilled in the art, and for example, may be derived from a natural product or may be obtained from sugar cane. Compounds derived from natural products can contribute to further minimizing irritation to the skin.

In addition, according to an embodiment, the preservative for external skin preparation may exhibit an excellent antimicrobial or preservative effect on various types of microorganisms (specifically, contaminated microorganisms). Specifically, the preservative for external skin preparation may inhibit or kill the growth of Gram-positive bacteria, Gram-negative bacteria, and molds (e.g., yeast, mold, etc.). Specifically, the preservative for external skin preparation may exhibit excellent antimicrobial or preservative effects on *Escherichia coli, Pseudomonas aeruginosa, Staphylococcus aureus, Candida albicans, Aspergillus brasiliensis,* and the like, but is not limited thereto.

In addition, according to an embodiment, the preservative for external skin preparation may exhibit excellent skin safety due to significantly lower skin irritation compared to the case of using a preservative mainly used in existing cosmetic products (a paraben preservative, imidazolidinylurea, phenoxyethanol, etc.), or octanediol, caprylglyceryl ether, or ethylhexylglycerin alone.

The preservative for external skin preparation may further include additives such as purified water, a carrier, an emulsifier, a moisturizer, a skin conditioning agent, a surfactant, a chelating agent, an antioxidant, an antiseptic agent, a germicide, a stabilizer, an additional preservative, a pH adjuster, a lubricant, a solubilizer, and a solvent. In addition, the preservative for external skin preparation may further include a substance capable of providing essential nutrients to the skin or a substance for improving skin conditions, such as a functional material having an anti-wrinkle or skin-whitening effect. In addition, the preservative for external skin preparation may further include an adjuvant including, but not limited to, a natural fragrance, a cosmetic fragrance, or a plant extract.

Another aspect provides a cosmetic composition including the preservative for external skin preparation. The terms or elements described in the description of the preservative for external skin preparation are the same as those already described above.

The cosmetic composition may be provided as a cosmetic composition having high preservative efficacy and stability by including the preservative for external skin preparation. An amount of the preservative for external skin preparation used in the cosmetic composition is not particularly limited as long as a desired preservative efficacy can be achieved, and may be appropriately determined by those skilled in the art according to the degree of the desired preservative efficacy.

In an embodiment, the preservative for external skin preparation may be included in an amount of 0.0001 to 50 wt% based on the total weight of the cosmetic composition. For example, the preservative may be included in an amount of 0.0001 to 0.001 wt%, 0.001 to 0.01 wt%, 0.01 to 0.1 wt%, 0.1 to 1 wt%, 1 to 10 wt%, 10 to 20 wt%, 20 to 30 wt%, 30 to 40 wt%, or 40 to 50 wt%. When an amount is out of these ranges, the antimicrobial or antiseptic effect may be reduced, and the deterioration of stability, such as solidification, precipitation, decrease in solubility, decrease in emulsification or solubilization, may be caused.

The cosmetic composition may further include an additive selected from the group consisting of purified water, a carrier, an emulsifier, a moisturizer, a skin conditioning agent, a surfactant, a chelating agent, an antioxidant, an antiseptic agent, a germicide, a stabilizer, a preservative, a pH adjuster, a lubricant, a solubilizer, a solvent, and a combination thereof, but is not limited thereto. In addition, the cosmetic composition may further include a substance capable of providing essential nutrients to the skin or a substance for improving skin conditions, such as a functional material having an anti-wrinkle or skin-whitening effect. The cosmetic composition may further include an adjuvant including, but not limited to, a natural scent, a cosmetic scent, or a plant extract.

The cosmetic composition may be prepared in any formulation conventionally prepared in the art. Specifically, the formulation of the cosmetic composition may be a solution, emulsion, suspension, softening lotion, nourishing lotion, massage cream, nourishing cream, pack, gel, skin adhesive type cosmetic, lipstick, powder, make-up base, foundation, shampoo, rinse, scalp cleanser, scalp lotion, scalp cream, scalp pack, scalp gel, scalp ointment, body cleanser, soap, toothpaste, mouthwash, paste, lotion, ointment, cream, patch, spray, or aerosol, but is not limited thereto.

The carrier included in the cosmetic composition may be selectively used depending on the formulation of the cosmetic. For example, when a cosmetic product in the form of an ointment, paste, cream, or gel is prepared, wax, paraffin, starch, tragacanth, cellulose derivatives, polyethylene glycol, silicone, bentonite, silica, talc, zinc oxide, and the like may be used alone or in combination as a carrier component. When a cosmetic product in the form of powder or spray is prepared, lactose, talc, silica, aluminum hydroxide, calcium silicate, polyamide powder, chlorofluorohydrocarbon, propane/butane, dimethyl ether, and the like may be used alone or in combination as a carrier component. When a cosmetic product in the form of a solution or emulsion is prepared, water, ethanol, isopropanol, ethyl carbonate, ethyl acetate, benzyl alcohol, benzyl benzoate, propylene glycol, 1,3-butylglycol oil, cottonseed oil, peanut oil, corn germ oil, olive oil, castor oil, sesame oil, glycerol aliphatic ester, polyethylene glycol, or fatty acid ester of sorbitan may be used alone or in combination as a carrier component. When a cosmetic product in the form of a suspension is prepared, water, ethanol or propylene glycol, ethoxylated isostearyl alcohol, polyoxyethylene sorbitol ester, polyoxyethylene sorbitan ester, microcrystalline cellulose, aluminum metahydroxide, bentonite, agar, tragacanth, and the like may be used alone or in combination as a carrier component. When a cosmetic product in the form of a soap is prepared, as a carrier component, an alkali metal salt of fatty acid, a fatty acid hemiester salt, fatty acid protein hydrolysate, isethionate, lanolin derivative, aliphatic alcohol, vegetable oil, glycerol, sugar, or the like may be used alone or in combination.

Another aspect provides a pharmaceutical composition for external skin preparation, the pharmaceutical composition including the preservative for external skin preparation. The terms or elements described in the description of the preservative for external skin preparation are the same as those already described above.

An amount of the preservative for external skin preparation used in the pharmaceutical composition for external skin preparation is not particularly limited as long as a desired preservative efficacy can be secured, and can be appropriately determined by those skilled in the art according to the degree of the desired preservative efficacy. For example, the preservative for external skin preparation may be included in an amount of 0.0001 to 50 wt% based on the total weight of the pharmaceutical composition for external skin preparation. For example, the preservative for external skin preparation may be included in an amount of 0.0001 to 0.001 wt%, 0.001 to 0.01 wt%, 0.01 to 0.1 wt%, 0.1 to 1 wt%, 1 to 10 wt%, 10 to 20 wt%, 20 to 30 wt%, 30 to 40 wt%, or 40 to 50 wt%.

The pharmaceutical composition for external skin preparation may be any formulation known as an external skin preparation, and may be, for example, one selected from the group consisting of a plaster, a liniment agent, an ointment, a pasta agent, a cataplasma agent, a suspending agent, an emulsion, a lotion, an aerosol agent, and a suppository, but is not limited thereto.

The pharmaceutical composition for external skin preparation may be for systemic action of an active ingredient by application to the skin or may be for topical action of an active ingredient by application to the skin. In an embodiment, the pharmaceutical composition may be for the topical action of an active ingredient by application to skin.

The cosmetic composition and the pharmaceutical composition for external skin preparation may further include various known additives in addition to the preservative for external skin preparation within a range that does not impair the effect of the preservative for external skin preparation according to the formulation, and according to an embodiment, the cosmetic composition and the pharmaceutical composition for external skin preparation may further include an additive selected from the group consisting of a carrier, an emulsifier, a moisturizer, a skin conditioning agent, a surfactant, a chelating agent, an antioxidant, a germicide, a stabilizer, and any combination thereof.

Still another aspect provides a method of preparing a cosmetic composition, the method including adding the preservative for external skin preparation.

The terms or elements described in the description of the preservative for external skin preparation and the cosmetic composition are the same as those already described above.

The preservative for external skin preparation may be used in an amount of 0.0001 to 50 wt% based on the cosmetic composition.

Still another aspect provides a method of performing antimicrobial, preservation, or antiseptic treatment on a cosmetic composition, the method including introducing a preservative for external skin preparation.

Another aspect provides use of a composition for external skin preparation or a cosmetic composition for antimicrobial, preservation, or antiseptic treatment, the composition including hinokitiol; and at least one solvent selected from the group consisting of alkanediol having 3 to 10 carbon atoms, alkanetriol having 3 to 6 carbon atoms, dipropylene glycol, glyceryl caprylate, ethylhexylglycerin, and phenoxyethanol.

**Advantageous Effects of disclosure**

The preservative for external skin preparation according to an aspect is remarkably excellent in antimicrobial power, antiseptic power, and safety, and thus, when applied to a cosmetic composition, it may be used as a skin-safe preservative, antiseptic, or antiseptic adjuvant.

In addition, the preservative for external skin preparation has excellent formulation stability, maintaining a liquid state without discoloration, off-odor, or precipitation under various temperature conditions, specifically, room temperature and refrigeration. Accordingly, it can be stably applied to cosmetic compositions by resolving stability issues that may otherwise occur due to the inclusion of hinokitiol and at least one solvent selected from the group consisting of alkanediol having 3 to 10 carbon atoms, alkanetriol having 3 to 6 carbon atoms, dipropylene glycol, glyceryl caprylate, ethylhexylglycerin, and phenoxyethanol. In addition, the preservative for external skin preparation has significantly lower skin irritation and thus can exhibit excellent skin safety, compared to the case of using the preservative (parabens preservative, imidazolidinylurea, phenoxyethanol, etc.) mainly used in existing cosmetic products, or alkanediol, alkanetriol, dipropylene glycol, glyceryl caprylate, or ethylhexylglycerin alone.

**Mode for Carrying out the disclosure**

Hereinafter, the present disclosure will be described in more detail through examples. However, these examples are intended to exemplarily explain the present disclosure, and the scope of the present disclosure is not limited to these examples.

**Experimental Example** 1: **Evaluation of Formulation Stability of Preservative Depending on Solvent Type**

In this experimental example, the formulation stability of the preservative was evaluated according to the solvent type, specifically with respect to whether a liquid state was maintained. To this end, preservatives were prepared according to the components and amounts shown in Table 1 and Table 2 below, and then stirred at room temperature.

[Table 1]

| Raw material name (Unit: wt%, w/w) | Example 1 | Example 2 | Example 3 | Example 4 | Example 5 | Example 6 | Example 7 | Example 8 | Example 9 | Example 10 | Example 11 | Example 12 | Example 13 | Example 14 | Example 15 | Example 16 |
|---|---|---|---|---|---|---|---|---|---|---|---|---|---|---|---|---|
| Hinokitiol | 3 | 3 | 3 | 3 | 3 | 3 | 3 | 3 | 3 | 3 | 3 | 3 | 3 | 3 | 3 | 3 |
| Glycerin | 97 | | | | | | | | | | | | | | | |
| 1,3-Propanediol | | 97 | | | | | | | | | | | | | | |
| Dipropylene Glycol | | | 97 | | | | | | | | | | | | | |
| Methylpropanediol | | | | 97 | | | | | | | | | | | | |
| 2,3-Butanediol | | | | | 97 | | | | | | | | | | | |
| 1,3-Butanediol | | | | | | 97 | | | | | | | | | | |
| 1,2-Pentanediol | | | | | | | 97 | | | | | | | | | |
| 1,5-Pentanediol | | | | | | | | 97 | | | | | | | | |
| 1,2-Hexanediol | | | | | | | | | 97 | | | | | | | |
| 1,2,6-Hexanetriol | | | | | | | | | | 97 | | | | | | |
| 1,2-Heptanediol | | | | | | | | | | | 97 | | | | | |
| 1,2-Octanediol | | | | | | | | | | | | 97 | | | | |
| 1,2-Decanediol | | | | | | | | | | | | | 97 | | | |
| Ethylhexylglycerin | | | | | | | | | | | | | | 97 | | |
| Glyceryl Caprylate | | | | | | | | | | | | | | | 97 | |
| Phenoxyethanol | | | | | | | | | | | | | | | | 97 |
| Room Temperature (25°C) for Evaluation | Liquid | Liquid | Liquid | Liquid | Liquid | Liquid | Liquid | Liquid | Liquid | Liquid | Liquid | Liquid | Liquid | Liquid | Liquid | Liquid |

[Table 2]

| Raw material name (Unit: wt%, w/w) | Comparative Example 1 | Comparative Example 2 | Comparative Example 3 |
|---|---|---|---|
| Hinokitiol | 3 | 3 | 3 |
| 1,6-Hexanediol | 97 | | |
| 1,10-Decanediol | | 97 | |
| Glyceryl Undecylenate | | | 97 |
| Room Temperature (25°C) for Evaluation | Solidified | Solidified | Solidified |

As a result, according to Tables 1 and 2, the preservatives (Examples 1 to 16) using alkanediol having 3 to 10 carbon atoms, alkanetriol having 3 to 6 carbon atoms, dipropylene glycol, glyceryl caprylate, ethylhexylglycerin, or phenoxyethanol maintained the liquid state at room temperature (25 °C), but Comparative Examples 1 to 3 were solidified.

**Experimental Example 2: Evaluation of Antimicrobial Power According to Solvent Type**

In the present experimental example, antimicrobial power of the preservatives against *Escherichia coli* (ATCC 8739), *Pseudomonas aeruginosa* (ATCC 9027), *Staphylococcus aureus* (ATCC 6538), *Candida albicans* (ATCC 10231), and *Aspergillus brasiliensis* (ATCC 16404) strains according to the type of solvent was evaluated. To this end, preservatives were prepared according to the components and amounts described in Table 3 and Table 4 below, and the minimum inhibitory concentration (MIC) method was performed. Specifically, the preservative was diluted to 0.0025%-5% (w/w) and prepared. At this time, the diluted solution was prepared by diluting with tryptic soy broth (TSB) for *Escherichia coli, Pseudomonas aeruginosa,* and *Staphylococcus aureus,* and potato dextrose broth (PDB) for *Candida albicans* and *Aspergillus brasiliensis,* and dispensed at 200 μL per well to 96 well plate. The strains dispensed in tryptic soy broth were seeded with a culture of the strains activated by culturing in a liquid medium at 37 °C under aerobic conditions for 18 hours so that the concentration was greater than 10^6 CFU/ml. The strains dispensed in PDB were seeded with strains activated by culturing at 25 °C under aerobic conditions so that the concentration was greater than 10^5 CFU/ml.

After that, the culture was performed according to each culture condition, and the absorbance was measured at 600 nm to confirm the MIC. The MIC refers to a minimum concentration (w/w %) of a sample capable of inhibiting the growth of the strain by about 95% or more.

[Table 3]

| Raw material name (Unit: wt%, w/w) | Example 17 | Example 18 | Example 19 | Example 20 | Example 21 | Example 22 | Example 23 | Example 24 | Example 25 | Example 26 | Example 27 | Example 28 | Example 29 | Example 30 | Example 31 | Example 32 |
|---|---|---|---|---|---|---|---|---|---|---|---|---|---|---|---|---|
| Hinokitiol | 1 | 1 | 1 | 1 | 1 | 1 | 1 | 1 | 1 | 1 | 1 | 1 | 1 | 1 | 1 | 1 |
| Glycerin | 99 | | | | | | | | | | | | | | | |
| 1,3-Propanediol | | 99 | | | | | | | | | | | | | | |
| Dipropylene Glycol | | | 99 | | | | | | | | | | | | | |
| Methylpropanediol | | | | 99 | | | | | | | | | | | | |
| 2,3-Butanediol | | | | | 99 | | | | | | | | | | | |
| 1,3-Butanediol | | | | | | 99 | | | | | | | | | | |
| 1,2-Pentanediol | | | | | | | 99 | | | | | | | | | |
| 1,5-Pentanediol | | | | | | | | 99 | | | | | | | | |
| 1,2-Hexanediol | | | | | | | | | 99 | | | | | | | |
| 1,2,6-Hexanetriol | | | | | | | | | | 99 | | | | | | |
| 1,2-Heptanediol | | | | | | | | | | | 99 | | | | | |
| 1,2-Octanediol | | | | | | | | | | | | 99 | | | | |
| 1,2-Decanediol | | | | | | | | | | | | | 99 | | | |
| Ethylhexylglycerin | | | | | | | | | | | | | | 99 | | |
| Glyceryl Caprylate | | | | | | | | | | | | | | | 99 | |
| Phenoxyethanol | | | | | | | | | | | | | | | | 99 |

[Table 4]

| Raw material name (Unit: wt%, w/w) | Comparative Example 4 | Comparative Example 5 | Comparative Example 6 |
|---|---|---|---|
| Hinokitiol | 1 | 1 | 1 |
| 1,6-Hexanediol | 99 | | |
| 1,10-Decanediol | | 99 | |
| Glyceryl Undecylenate | | | 99 |

[Table 5]

| Strain | Ex.17 | Ex.18 | Ex.19 | Ex.20 | Ex.21 | Ex.22 | Ex.23 | Ex.24 | Ex.25 | Ex.26 | Ex.27 | Ex.28 | Ex.29 | Ex.30 | Ex.31 | Ex.32 | Comp. Ex.4 | Comp. Ex.5 | Comp. Ex.6 |
|---|---|---|---|---|---|---|---|---|---|---|---|---|---|---|---|---|---|---|---|
| E. coli | 0.3125 | 0.3125 | 0.3125 | 0.3125 | 0.3125 | 0.3125 | 0.3125 | 0.3125 | 0.156 | 0.3125 | 0.3125 | 0.156 | 0.3125 | 0.156 | 0.156 | 0.078 | >5 | >5 | >5 |
| P. aeruginosa | 2.5 | 2.5 | 2.5 | 2.5 | 2.5 | 2.5 | 2.5 | 2.5 | 1.25 | 2.5 | 2.5 | 1.25 | 2.5 | 1.25 | 1.25 | 0.625 | >5 | >5 | >5 |
| S. aureus | 0.156 | 0.156 | 0.156 | 0.156 | 0.156 | 0.156 | 0.156 | 0.156 | 0.156 | 0.156 | 0.156 | 0.156 | 0.156 | 0.156 | 0.156 | 0.078 | >5 | >5 | >5 |
| C. albicans | 0.04 | 0.04 | 0.04 | 0.04 | 0.04 | 0.04 | 0.04 | 0.04 | 0.04 | 0.04 | 0.04 | 0.04 | 0.04 | 0.04 | 0.04 | 0.04 | >5 | >5 | >5 |
| A. brasiliensis | 0.078 | 0.078 | 0.078 | 0.078 | 0.078 | 0.078 | 0.078 | 0.078 | 0.078 | 0.078 | 0.078 | 0.078 | 0.078 | 0.078 | 0.078 | 0.078 | >5 | >5 | >5 |

Unit(%) As a result, as shown in Table 5, the preservatives (Examples 17 to 32) using alkanediol having 3 to 10 carbon atoms, alkanetriol having 3 to 6 carbon atoms, dipropylene glycol, glyceryl caprylate, ethylhexylglycerin, or phenoxyethanol showed excellent antimicrobial power due to significantly lower MIC than Comparative Examples 4 to 6.

Referring to the results of Experimental Examples 1 and 2, it can be seen that the preservatives including hinokitiol have excellent formulation stability and antimicrobial power when alkanediol having 3 to 10 carbon atoms, alkanetriol having 3 to 6 carbon atoms, dipropylene glycol, glyceryl caprylate, ethylhexylglycerin, or phenoxyethanol is used as a solvent.

**Experimental Example 3: Evaluation of Formulation Stability Depending on Weight Ratio of Preservative Component**

In this experimental example, the formulation stability of the preservative was evaluated according to the solvent weight ratio to hinokitiol, specifically with respect to whether a liquid state was maintained. To this end, preservatives were prepared according to the components and amounts shown in Table 6 and Table 7 below, and then stirred at room temperature.

[Table 6]

| Raw material name (Unit: wt%, w/w) | Example 33 | Example 34 | Example 35 | Example 36 | Example 37 | Example 38 | Example 39 | Example 40 | Example 41 | Example 42 | Example 43 | Example 44 | Example 45 | Example 46 | Example 47 | Example 48 |
|---|---|---|---|---|---|---|---|---|---|---|---|---|---|---|---|---|
| Hinokitiol | 5 | 5 | 5 | 5 | 5 | 5 | 5 | 5 | 5 | 5 | 5 | 5 | 5 | 5 | 5 | 5 |
| Glycerin | 95 | | | | | | | | | | | | | | | |
| 1,3-Propanediol | | 95 | | | | | | | | | | | | | | |
| Dipropylene Glycol | | | 95 | | | | | | | | | | | | | |
| Methylpropanediol | | | | 95 | | | | | | | | | | | | |
| 2,3-Butanediol | | | | | 95 | | | | | | | | | | | |
| 1,3-Butanediol | | | | | | 95 | | | | | | | | | | |
| 1,2-Pentanediol | | | | | | | 95 | | | | | | | | | |
| 1,5-Pentanediol | | | | | | | | 95 | | | | | | | | |
| 1,2-Hexanediol | | | | | | | | | 95 | | | | | | | |
| 1,2,6-Hexanetriol | | | | | | | | | | 95 | | | | | | |
| 1,2-Heptanediol | | | | | | | | | | | 95 | | | | | |
| 1,2-Octanediol | | | | | | | | | | | | 95 | | | | |
| 1,2-Decanediol | | | | | | | | | | | | | 95 | | | |
| Ethylhexylglycerin | | | | | | | | | | | | | | 95 | | |
| Glyceryl Caprylate | | | | | | | | | | | | | | | 95 | |
| Phenoxyethanol | | | | | | | | | | | | | | | | 95 |
| Room temperature (25°C) | Liquid | Liquid | Liquid | Liquid | Liquid | Liquid | Liquid | Liquid | Liquid | Liquid | Liquid | Liquid | Liquid | Liquid | Liquid | Liquid |

[Table 7]

| Raw material name (Unit: wt%, w/w) | Comp. Ex. 7 | Comp. Ex. 8 | Comp. Ex.9 | Comp. Ex.10 | Comp. Ex.11 | Comp. Ex.12 | Comp. Ex.13 | Comp. Ex.14 | Comp. Ex.15 | Comp. Ex.16 | Comp. Ex. 17 | Comp. Ex.18 | Comp. Ex.19 | Comp. Ex.20 | Comp. Ex.21 | Comp. Ex.22 |
|---|---|---|---|---|---|---|---|---|---|---|---|---|---|---|---|---|
| Hinokitiol | 7 | 7 | 7 | 7 | 7 | 7 | 7 | 7 | 7 | 7 | 7 | 7 | 7 | 7 | 7 | 7 |
| Glycerin | 93 | | | | | | | | | | | | | | | |
| 1,3-Propanediol | | 93 | | | | | | | | | | | | | | |
| Dipropylene Glycol | | | 93 | | | | | | | | | | | | | |
| Methylpropanediol | | | | 93 | | | | | | | | | | | | |
| 2,3-Butanediol | | | | | 93 | | | | | | | | | | | |
| 1,3-Butanediol | | | | | | 93 | | | | | | | | | | |
| 1,2-Pentanediol | | | | | | | 93 | | | | | | | | | |
| 1,5-Pentanediol | | | | | | | | 93 | | | | | | | | |
| 1,2-Hexanediol | | | | | | | | | 93 | | | | | | | |
| 1,2,6-Hexanetriol | | | | | | | | | | 93 | | | | | | |
| 1,2-Heptanediol | | | | | | | | | | | 93 | | | | | |
| 1,2-Octanediol | | | | | | | | | | | | 93 | | | | |
| 1,2-Decanediol | | | | | | | | | | | | | 93 | | | |
| Ethylhexyl glycerin | | | | | | | | | | | | | | 93 | | |
| Glyceryl Caprylate | | | | | | | | | | | | | | | 93 | |
| Phenoxyethanol | | | | | | | | | | | | | | | | 93 |
| Room temperature (25°C) | Ppt. | Ppt. | Ppt. | Ppt. | Ppt. | Ppt. | Ppt. | Ppt. | Ppt. | Ppt. | Ppt. | Ppt. | Ppt. | Ppt. | Ppt. | Ppt. |

As a result, according to Table 6 and Table 7, it was confirmed that the preservatives including hinokitiol and the solvent in a weight ratio of 1:19 (Examples 33 to 48) maintained a liquid state at room temperature, whereas precipitation (Ppt.) occurred in the preservatives including hinokitiol and the solvent in a weight ratio of less than 1:19 (Comparative Examples 7 to 22).

**Experimental Example 4: Evaluation of Antimicrobial Power According to Weight Ratio of Preservative Component**

In this experimental example, the antimicrobial power against strains according to the solvent weight ratio to hinokitiol was evaluated. To this end, preservatives were prepared according to the components and amounts of Examples (Examples 33 to 48) and Comparative Examples (Comparative Examples 7 to 22) in Experimental Example 3, and the MIC method was performed in the same manner as in Experimental Example 2.

[Table 8]

| Strain | Example 33 | Example 34 | Example 35 | Example 36 | Example 37 | Example 38 | Example 39 | Example 40 | Example 41 | Example42 | Example 43 | Example 44 | Example 45 | Exam ple 46 | Example 47 | Example 48 |
|---|---|---|---|---|---|---|---|---|---|---|---|---|---|---|---|---|
| E. coli | 0.0625 | 0.0625 | 0.0625 | 0.0625 | 0.0625 | 0.0625 | 0.0625 | 0.0625 | 0.0625 | 0.0625 | 0.0625 | 0.0625 | 0.0625 | 0.0625 | 0.0625 | 0.02 |
| P. aeruginosa | 0.5 | 0.5 | 0.5 | 0.5 | 0.5 | 0.5 | 0.5 | 0.5 | 0.25 | 0.5 | 0.5 | 0.5 | 0.5 | 0.25 | 0.25 | 0.125 |
| S. aureus | 0.04 | 0.04 | 0.04 | 0.04 | 0.04 | 0.04 | 0.04 | 0.04 | 0.04 | 0.04 | 0.04 | 0.04 | 0.04 | 0.04 | 0.04 | 0.02 |
| C. albicans | 0.01 | 0.01 | 0.01 | 0.01 | 0.01 | 0.01 | 0.01 | 0.01 | 0.01 | 0.01 | 0.01 | 0.01 | 0.01 | 0.01 | 0.01 | 0.01 |
| A. brasiliensis | 0.02 | 0.02 | 0.02 | 0.02 | 0.02 | 0.02 | 0.02 | 0.02 | 0.02 | 0.02 | 0.02 | 0.02 | 0.02 | 0.02 | 0.02 | 0.02 |
| Unit(%) | | | | | | | | | | | | | | | | |

[Table 9]

| Strain | Comp. Ex.7 | Comp. Ex.8 | Comp. Ex.9 | Comp. Ex.10 | Comp. Ex.11 | Comp. Ex.12 | Comp. Ex.13 | Comp. Ex.14 | Comp. Ex.15 | Comp. Ex.16 | Comp. Ex.17 | Comp. Ex.18 | Comp. Ex.19 | Comp. Ex.20 | Comp. Ex.21 | Comp. Ex.22 |
|---|---|---|---|---|---|---|---|---|---|---|---|---|---|---|---|---|
| E. coli | >5 | >5 | >5 | >5 | >5 | >5 | >5 | >5 | >5 | >5 | >5 | >5 | >5 | >5 | >5 | >5 |
| P. aeruginosa | >5 | >5 | >5 | >5 | >5 | >5 | >5 | >5 | >5 | >5 | >5 | >5 | >5 | >5 | >5 | >5 |
| S. aureus | >5 | >5 | >5 | >5 | >5 | >5 | >5 | >5 | >5 | >5 | >5 | >5 | >5 | >5 | >5 | >5 |
| C. albicans | >5 | >5 | >5 | >5 | >5 | >5 | >5 | >5 | >5 | >5 | >5 | >5 | >5 | >5 | >5 | >5 |
| A. brasiliensis | >5 | >5 | >5 | >5 | >5 | >5 | >5 | >5 | >5 | >5 | >5 | >5 | >5 | >5 | >5 | >5 |
| Unit(%) | | | | | | | | | | | | | | | | |

As a result, as shown in Table 8 and Table 9, in the case of the preservatives including hinokitiol and the solvent in a weight ratio of 1:19 (Examples 33 to 48), the MIC was significantly lower compared to the preservatives including hinokitiol and the solvent in a weight ratio of less than 1:19 (Comparative Examples 4 to 19), thereby showing excellent antimicrobial activity.

When the results of Experimental Examples 3 and 4 were summarized, it was confirmed that in the case of the preservatives including hinokitiol, when the weight ratio of hinokitiol and the solvent was less than 1:19, the formulation stability and antimicrobial power were decreased.

**Experimental Example 5: Evaluation of Antimicrobial Synergistic Effect Depending on Solvent Type**

In the present experimental example, the synergistic effect with the antimicrobial power of hinokitiol according to the type of solvent was evaluated in *Escherichia coli* (ATCC 8739), *Pseudomonas aeruginosa* (ATCC 9027), *Staphylococcus aureus* (ATCC 6538), *Candida albicans* (ATCC 10231), and *Aspergillus brasiliensis* (ATCC 16404). To this end, the fractional inhibition concentration (FIC) index was calculated by a checkerboard test.

Specifically, 100 $\mu$l of TSB or PDB medium, 50 $\mu$l of hinokitiol (0.04-2.5%), and 50 $\mu$l of a solvent were dispensed into a 96-well plate to obtain the concentrations shown below, and the total volume was adjusted to 200 $\mu$l.

Glycerin, 1,3-propanediol, dipropylene glycol, methylpropanediol, 2,3-butanediol, and 1,3-butanediol were diluted to 1.25-40% (w/w), 1,2-pentanediol, 1,5-pentanediol, 1,2-hexanediol, 1,2,6-hexanetriol, and 1,2-heptanediol were diluted to 0.125-4% (w/w), 1,2-octanediol, 1,2-decanediol, ethylhexylglycerin, glyceryl caprylate, and phenoxyethanol were diluted to 0.03-1% (w/w). The strain culture activated by incubation in liquid medium for 18 hours was seeded to be >10^ 6CFU/ml for *Escherichia coli, Pseudomonas aeruginosa,* and *Staphylococcus aureus,* and to be >10^ 5 CFU/ml for *Candida albicans* and *Aspergillus brasiliensis.* After that, the culture was performed according to each culture condition, and the absorbance was measured at 600 nm to confirm the MIC. Thereafter, the FIC index was calculated by Equation 1 below. An FIC index of 0.5 or less indicates a synergistic effect; more than 0.5 to 1.0 indicates an additive effect; more than 1.0 to 4.0 indicates an indifferent effect; and more than 4.0 indicates an antagonistic effect.

[Equation 1]

$$\text{FIC index} = \text{FICa} + \text{FICb}$$

(FICa = MICa in combination / MICa, FICb = MICb in combination / MICb)

[Table 10]

| Raw Material Name | FIC Index Results | | | | |
|---|---|---|---|---|---|
| | E. coli | P. aeruginosa | S. aureus | C. albicans | A. brasiliensis |
| Glycerin | 0 | + | 0 | 0 | + |
| 1,3-Propanediol | 0 | + | 0 | 0 | + |
| Dipropylene Glycol | 0 | + | 0 | 0 | + |
| Methylpropanediol | 0 | + | 0 | 0 | + |
| 2,3-Butanediol | 0 | + | 0 | 0 | + |
| 1,3-Butanediol | 0 | + | 0 | 0 | + |
| 1,2-Pentanediol | 0 | ++ | 0 | + | + |
| 1,5-Pentanediol | 0 | ++ | 0 | + | + |
| 1,2-Hexanediol | 0 | + | 0 | 0 | + |
| 1,2,6-Hexanetriol | 0 | + | 0 | 0 | + |
| 1,2-Heptanediol | 0 | + | 0 | 0 | + |
| 1,2-Octanediol | 0 | + | 0 | + | ++ |
| 1,2-Decanediol | 0 | + | 0 | + | + |
| Ethylhexylglycerin | 0 | + | 0 | + | + |
| Glyceryl Caprylate | 0 | + | 0 | + | + |
| Phenoxyethanol | 0 | + | 0 | 0 | + |

(continued)

| Raw Material Name | FIC Index Results | | | | |
|---|---|---|---|---|---|
| | E. coli | P. aeruginosa | S. aureus | C. albicans | A. brasiliensis |
| 1,6-Hexanediol | - | 0 | 0 | 0 | 0 |
| 1,10-Decanediol | 0 | 0 | 0 | 0 | 0 |
| Glyceryl Undecylenate | 0 | 0 | 0 | 0 | 0 |
| -: offset, 0: irrelevant, +: addition, ++: synergy | | | | | |

As a result, as shown in Table 10, when alkanediol having 3 to 10 carbon atoms, alkanetriol having 3 to 6 carbon atoms, dipropylene glycol, glyceryl caprylate, ethylhexylglycerin, or the phenoxyethanol was used, an addition or synergy effect with hinokitiol was shown in at least one strain. On the other hand, when the solvent was not used, it was confirmed that irrelevant effects or offset effects were exhibited in all strains. In particular, 1,2-pentanediol, 1,5-pentanediol, 1,2-octanediol, and glyceryl caprylate exhibited a synergistic effect with hinokitiol.

As a result, it was confirmed that an antimicrobial synergistic effect with hinokitiol appeared when a specific solvent was used.

**Experimental Example 6: Preparation of Composition for External Skin Preparation and Evaluation of Antiseptic Power**

In this experimental example, after preparing a composition for external skin preparation containing a preservative, a certain number of bacteria were seeded, and then the number of viable bacteria remaining was checked to evaluate the antiseptic power.

First, preservatives were prepared with the composition of Table 11 to prepare a composition for external skin preparation. The preservatives of Formulation Examples 1 to 12 include both hinokitiol and the solvent, and the preservative of Formulation Example 13 includes hinokitiol as a sole ingredient.

Then, phase A to phase D were prepared with the components and amounts shown in Table 12, and the prepared phases were mixed with 1 wt% of a preservative (Formulation Examples 1 to 12) or 0.05 wt% of hinokitiol as a sole ingredient (Formulation Example 13) to prepare compositions for external skin preparation.

[Table 11]

| | Raw Material | Weight Ratio (Hinokitiol : Solvent) |
|---|---|---|
| Formulation Example 1 | Hinokitiol; 1,3-Propanediol | 5 : 95 |
| Formulation Example 2 | Hinokitiol; 1,2-Octanediol | 5 : 95 |
| Formulation Example 3 | Hinokitiol; 1,2,6-Hexanetriol | 5 : 95 |
| Formulation Example 4 | Hinokitiol; Ethylhexylglycerin | 5 : 95 |
| Formulation Example 5 | Hinokitiol; Glyceryl Caprylate | 5 : 95 |
| Formulation Example 6 | Hinokitiol; 1,3-Propanediol | 7 : 93 |
| Formulation Example 7 | Hinokitiol; 1,2-Octanediol | 7 : 93 |
| Formulation Example 8 | Hinokitiol; 1,2,6-Hexanetriol | 7 : 93 |
| Formulation Example 9 | Hinokitiol; Ethylhexylglycerin | 7 : 93 |
| Formulation Example 10 | Hinokitiol; Glyceryl Caprylate | 7 : 93 |
| Formulation Example 11 | Hinokitiol; 1,10-Decanediol | 5 : 95 |
| Formulation Example 12 | Hinokitiol; Glyceryl Undecylenate | 5 : 95 |
| Formulation Example 13 | Hinokitiol alone | - |

[Table 12]

|  | | Ingredient | Amount (w/w) |
|---|---|---|---|
| Phase A | | Distilled Water | Up to 100 |
| | | Glycerin | 3 |
| | | Sodium Chlorate | 0.9 |
| | | Polysorbate 20 | 0.9 |
| Phase B | | Methyltrimethicone | 23 |
| | | Dimethicone | 4.5 |
| | | Propylene Glycol Dicaprate | 2 |
| | | PEG-10 Dimethicone | 3 |
| Phase C | | Titanium Dioxide | 10.5 |
| | | Polyglyceryl-2 Triisostearate | 2.5 |
| Phase D | | Dimethicone | 8.7 |
| | | Silica | 4.5 |

Thereafter, in order to evaluate the antiseptic power of the composition for external skin preparation, in the case of bacteria, *Escherichia coli, Pseudomonas aeruginosa,* and *Staphylococcus aureus* were mixed at the same concentration in 1:1:1 and seeded so that the initial concentration of bacteria per sample was about 10^6 CFU/g. For yeast and molds, *Candida albicans* and *Aspergillus brasiliensis* were seeded to an initial concentration of about 10^5 CFU/g, respectively. Thereafter, while the sample was stored at room temperature (25°C) for 4 weeks, 1 g of each sample was taken at 7-day intervals to check the number of remaining probiotics. In particular, in the case of molds, the presence or absence of off-odor and the presence or absence of hyphae and spores on the surface of the samples were observed.

[Table 13]

| CFU/g | Bacteria | | | | Yeast | | | | Mold | | | |
|---|---|---|---|---|---|---|---|---|---|---|---|---|
| | Week 1 | Week 2 | Week 3 | Week 4 | Week 1 | Week 2 | Week 3 | Week 4 | Week 1 | Week 2 | Week 3 | Week 4 |
| Formulation Example 1 | <1000 | <100 | <10 | <10 | <100 | <100 | <10 | <10 | - | - | - | - |
| Formulation Example 2 | <10 | <10 | <10 | <10 | <10 | <10 | <10 | <10 | - | - | - | - |
| Formulation Example 3 | <1000 | <10 | <10 | <10 | <100 | <10 | <10 | <10 | - | - | - | - |
| Formulation Example 4 | <10 | <10 | <10 | <10 | <10 | <10 | <10 | <10 | - | - | - | - |
| Formulation Example 5 | <1000 | <10 | <10 | <10 | <10 | <10 | <10 | <10 | - | - | - | - |
| Formulation Example 6 | $<10^4$ | $<10^4$ | $<10^5$ | $<10^5$ | $<10^4$ | $<10^4$ | <1000 | <1000 | - | - | + | ++ |
| Formulation Example 7 | $<10^4$ | $<10^4$ | $<10^4$ | $<10^5$ | $<10^4$ | $<10^4$ | <1000 | <1000 | - | + | ++ | ++ |
| Formulation Example 8 | $<10^4$ | $<10^4$ | $<10^4$ | $<10^5$ | <1000 | <1000 | <1000 | <1000 | - | - | + | ++ |
| Formulation Example 9 | $<10^4$ | $<10^4$ | $<10^4$ | $<10^5$ | <1000 | <1000 | <100 | <100 | - | + | ++ | ++ |
| Formulation Example 10 | $<10^4$ | $<10^4$ | $<10^4$ | $<10^5$ | <1000 | <1000 | <100 | <100 | - | + | ++ | ++ |
| Formulation Example 11 | $<10^6$ | $<10^6$ | $<10^7$ | $<10^7$ | <1000 | <1000 | <100 | <100 | - | - | ++ | ++ |
| Formulation Example 12 | $<10^6$ | $<10^6$ | $<10^7$ | $<10^7$ | <1000 | <1000 | <100 | <100 | - | - | ++ | ++ |
| Formulation Example 13 | $<10^4$ | $<10^4$ | <1000 | <1000 | <100 | <100 | <10 | <10 | - | - | ++ | ++ |

-: Normal state with no occurrence of off-odor, hyphae, or spores; +: Mold growth observed on a portion of the sample surface; ++: Occurrence of off-odor and mold growth on the sample surface.

As a result, as shown in Table 13, when the weight ratio of hinokitiol and the solvent was 1:19 or more (Formulation Examples 1 to 5), the number of bacteria, yeast, and molds were all significantly reduced compared to the case of less than 1:19 (Formulation Examples 6 to 10). On the other hand, in the compositions for external skin preparation (formulation examples 11 to 13) containing a preservative including 1,6-hexanediol, 1,10-decanediol, and glyceryl undecylenate, the number of bacteria and yeasts did not decrease or mold was generated on the surface of the sample. In addition, it was confirmed that the antiseptic power was significantly reduced in Formulation Example 13 including a preservative containing only hinokitiol without a solvent.

Therefore, when the above results are summarized, it can be seen that the antiseptic power of the preservative including hinokitiol and the solvent is determined by the type of the solvent and the weight ratio of hinokitiol and the solvent.

In addition, it could be seen that the specific solvent having antimicrobial power exhibited an antimicrobial synergistic effect with hinokitiol.

**Experimental Example 7: Preparation of Composition for External Skin Preparation Addtionally Including Caprylhydroxamic Acid or Raspberry Ketone and Evaluation of Antiseptic Power**

In the present experimental example, a composition for external skin preparation additionally including caprylhydroxamic acid or raspberry ketone was prepared, a certain number of bacteria were seeded, and then the remaining viable bacteria were checked to evaluate the antiseptic power.

First, preservatives were prepared with the composition of Table 14 to prepare a composition for external skin preparation. The preservatives of Examples 49 to 58 include all of hinokitiol, a solvent, and caprylhydroxamic acid or raspberry ketone, and the preservatives of Comparative Examples 23 to 24 include caprylhydroxamic acid or raspberry ketone as a sole ingredient.

Then, phase A to phase D were prepared with the components and amounts of Table 15, and were mixed with 1 wt% of a preservative (Examples 49 to 58) or 0.1 wt% of caprylhydroxamic acid or raspberry ketone as a sole ingredient (Comparative Examples 23 to 24) to prepare a composition for external skin preparation.

[Table 14]

| | | Raw Material | Weight Ratio (Hinokitiol : Solvent : Caprylhydroxamic Acid or Raspberry Ketone) |
|---|---|---|---|
| | Example 49 | Hinokitiol; 1,3-Propanediol; and Caprylhydroxamic Acid | 5 : 85 : 10 |
| | Example 50 | Hinokitiol; 1,2-Octanediol; and Caprylhydroxamic Acid | 5 : 85 : 10 |
| | Example 51 | Hinokitiol; 1,2,6-Hexanetriol; and Caprylhydroxamic Acid | 5 : 85 : 10 |
| | Example 52 | Hinokitiol; Ethylhexylglycerin; and Caprylhydroxamic Acid | 5 : 85 : 10 |
| | Example 53 | Hinokitiol; Glyceryl Caprylate; and Caprylhydroxamic Acid | 5 : 85 : 10 |
| | Example 54 | Hinokitiol; 1,3-Propanediol; and Raspberry Ketone | 5: 85 : 10 |
| | Example 55 | Hinokitiol; 1,2-Octanediol; and Raspberry Ketone | 5 : 85 : 10 |
| | Example 56 | Hinokitiol; 1,2,6-Hexanetriol; and Raspberry Ketone | 5: 85 : 10 |
| | Example 57 | Hinokitiol; Ethylhexylglycerin; and Raspberry Ketone | 5 : 85 : 10 |
| | Example 58 | Hinokitiol; Glyceryl Caprylate; and Raspberry Ketone | 5: 85 : 10 |
| | Comparative Example 23 | Caprylhydroxamic Acid alone | - |
| | Comparative Example 24 | Raspberry Ketone alone | - |

[Table 15]

| | Ingredient | Amount (w/w) |
|---|---|---|
| | Distilled Water | Up to 100 |
| Phase A | Glycerin | 3 |
| | Sodium Chlorate | 0.9 |
| | Polysorbate 20 | 0.9 |

(continued)

| | Ingredient | Amount (w/w) |
|---|---|---|
| Phase B | Methyltrimethicone | 23 |
| | Dimethicone | 4.5 |
| | Propylene Glycol Dicaprate | 2 |
| | PEG-10 Dimethicone | 3 |
| Phase C | Titanium Dioxide | 10.5 |
| | Polyglyceryl-2 Triisostearate | 2.5 |
| Phase D | Dimethicone | 8.7 |
| | Silica | 4.5 |

Thereafter, in order to evaluate the antiseptic power of the composition for external skin preparation, in the case of bacteria, Escherichia coli, Pseudomonas aeruginosa, and Staphylococcus aureus were mixed at the same concentration in 1:1:1 and seeded so that the initial concentration of bacteria per sample was about 10^6 CFU/g. For yeast and molds, *Candida albicans* and *Aspergillus brasiliensis* were seeded to an initial concentration of about 10^5 CFU/g, respectively. Thereafter, while the sample was stored at room temperature (25°C) for 4 weeks, 1 g of each sample was taken at 7-day intervals to check the number of remaining probiotics. In particular, in the case of molds, the presence or absence of off-odor and the presence or absence of hyphae and spores on the sample surface were checked for the sample.

[Table 16]

| CFU/g | Bacteria | | | | Yeast | | | | Mold | | | |
|---|---|---|---|---|---|---|---|---|---|---|---|---|
| | Week 1 | Week 2 | Week 3 | Week 4 | Week 1 | Week 2 | Week 3 | Week 4 | Week 1 | Week 2 | Week 3 | Week 4 |
| Example 49 | <10 | <10 | <10 | <10 | <10 | <10 | <10 | <10 | - | - | - | - |
| Example 50 | <10 | <10 | <10 | <10 | <10 | <10 | <10 | <10 | - | - | - | - |
| Example 51 | <10 | <10 | <10 | <10 | <10 | <10 | <10 | <10 | - | - | - | - |
| Example 52 | <10 | <10 | <10 | <10 | <10 | <10 | <10 | <10 | - | - | - | - |
| Example 53 | <10 | <10 | <10 | <10 | <10 | <10 | <10 | <10 | - | - | - | - |
| Example 54 | <10 | <10 | <10 | <10 | <10 | <10 | <10 | <10 | - | - | - | - |
| Example 55 | <10 | <10 | <10 | <10 | <10 | <10 | <10 | <10 | - | - | - | - |
| Example 56 | <10 | <10 | <10 | <10 | <10 | <10 | <10 | <10 | - | - | - | - |
| Example 57 | <10 | <10 | <10 | <10 | <10 | <10 | <10 | <10 | - | - | - | - |
| Example 58 | <10 | <10 | <10 | <10 | <10 | <10 | <10 | <10 | - | - | - | - |
| Comparative Example 23 | <1000 | <1000 | <100 | <100 | <1000 | <1000 | <100 | <100 | + | ++ | ++ | ++ |
| Comparative Example 24 | <1000 | <1000 | <100 | <100 | <1000 | <1000 | <100 | <100 | ++ | ++ | ++ | ++ |

-: Normal state with no occurrence of off-odor, hyphae, or spores; +: Mold growth observed on a portion of the sample surface; ++: Occurrence of off-odor and mold growth on the sample surface.

As a result, as shown in Table 16, it was confirmed that when all of hinokitiol, the solvent, and the caprylhydroxamic acid or raspberry ketone were included in the preservative (Examples 49 to 58), the number of bacteria and yeasts was significantly reduced, and no mold growth was observed on the surfaces of the samples, thereby confirming excellent antiseptic power. On the other hand, in Comparative Examples 23 to 24 including caprylhydroxamic acid or raspberry ketone alone as a sole ingredient for a preservative, the reduction in the number of bacteria and yeasts was insignificant, and mold growth was observed on the sample surfaces, thereby indicating a significantly reduced preservative efficacy.

In addition, in Examples 49 and 54, in which caprylhydroxamic acid or raspberry ketone was added to Formulation Example 1 of Experimental Example 6, a greater reduction in the numbers of bacteria and yeasts was observed at week 1 and week 2 than in Formulation Example 1. In addition, in Examples 51 and 56, in which caprylhydroxamic acid or raspberry ketone was added to Formulation Example 3 of Experimental Example 6, a greater reduction in the numbers of bacteria and yeasts was observed after 1 week than in Formulation Example 1. In addition, in Examples 53 and 58, in which caprylhydroxamic acid or raspberry ketone was added to Formulation Example 5 of Experimental Example 6, a greater reduction in the numbers of bacteria was observed after 1 week than in Formulation Example 1.

As a result, it was confirmed that when caprylhydroxamic acid or raspberry ketone was further included in the preservative, an antiseptic synergistic effect with hinokitiol and a solvent was exhibited.

**Experimental Example 8: Evaluation of Antiseptic Power Depending on Amount of Caprylhydroxamic Acid**

In the present experimental example, the antiseptic power was evaluated according to an amount of caprylhydroxamic acid.

To this end, preservatives were prepared by varying the weight ratio in the composition shown in Table 17 below. The preservative of each formulation example was prepared by maintaining the weight ratio of hinokitiol:solvent and caprylhydroxamic acid at 1:19 while varying an amount of caprylhydroxamic acid.

Then, phase A to phase D were prepared with the components and amounts of Table 18, and mixed with 1 wt% of a preservative (Formulation Examples 14 to 20) to prepare a composition for external skin preparation.

[Table 17]

|  | Raw Material | Weight Ratio (Hinokitiol : Solvent : Caprylhydroxamic Acid) |
|---|---|---|
| Formulation Example 14 | Hinokitiol; 1,3-Propanediol; and Caprylhydroxamic Acid | 5 : 94 : 1 |
| Formulation Example 15 | Hinokitiol; 1,3-Propanediol; and Caprylhydroxamic Acid | 5 : 92 : 3 |
| Formulation Example 16 | Hinokitiol; 1,3-Propanediol; and Caprylhydroxamic Acid | 5: 90 : 5 |
| Formulation Example 17 | Hinokitiol; 1,3-Propanediol; and Caprylhydroxamic Acid | 5 : 85 : 10 |
| Formulation Example 18 | Hinokitiol; 1,3-Propanediol; and Caprylhydroxamic Acid | 5 : 80 : 15 |
| Formulation Example 19 | Hinokitiol; 1,3-Propanediol; and Caprylhydroxamic Acid | 5 : 94.9 : 0.1 |
| Formulation Example 20 | Hinokitiol; 1,3-Propanediol; and Caprylhydroxamic Acid | 5 : 75 : 20 |

[Table 18]

|  | Ingredient | Amount (w/w) |
|---|---|---|
| Phase A | Distilled Water | Up to 100 |
|  | Glycerin | 3 |
|  | Sodium Chlorate | 0.9 |
|  | Polysorbate 20 | 0.9 |
| Phase B | Methyltrimethicone | 23 |
|  | Dimethicone | 4.5 |
|  | Propylene Glycol Dicaprate | 2 |
|  | PEG-10 Dimethicone | 3 |
| Phase C | Titanium Dioxide | 10.5 |
|  | Polyglyceryl-2 Triisostearate | 2.5 |
| Phase D | Dimethicone | 8.7 |
|  | Silica | 4.5 |

Thereafter, in order to evaluate the antiseptic power of the composition for external skin preparation, in the case of bacteria, *Escherichia coli, Pseudomonas aeruginosa,* and *Staphylococcus aureus* were mixed at the same concentration in 1:1:1 and seeded so that the initial concentration of bacteria per sample was about 10^6 CFU/g. For yeast and molds, *Candida albicans* and *Aspergillus brasiliensis* were seeded to an initial concentration of about 10^5 CFU/g, respectively. Thereafter, while the sample was stored at room temperature (25°C) for 4 weeks, 1 g of each sample was taken at 7-day intervals to check the number of remaining probiotics. In particular, in the case of molds, the presence or absence of off-odor and the presence or absence of hyphae and spores on the sample surface were checked for the sample.

[Table 19]

| CFU/g | Bacteria | | | | Yeast | | | | Mold | | | |
|---|---|---|---|---|---|---|---|---|---|---|---|---|
| | Week 1 | Week 2 | Week 3 | Week 4 | Week 1 | Week 2 | Week 3 | Week 4 | Week 1 | Week 2 | Week 3 | Week 4 |
| Formulation Example 14 | <100 | <100 | <10 | <10 | <10 | <10 | <10 | <10 | - | - | - | + |
| Formulation Example 15 | <100 | <10 | <10 | <10 | <10 | <10 | <10 | <10 | - | - | - | + |
| Formulation Example 16 | <100 | <10 | <10 | <10 | <10 | <10 | <10 | <10 | - | - | - | - |
| Formulation Example 17 | <10 | <10 | <10 | <10 | <10 | <10 | <10 | <10 | - | - | - | - |
| Formulation Example 18 | <10 | <10 | <10 | <10 | <10 | <10 | <10 | <10 | - | - | - | - |
| Formulation Example 19 | <1000 | <100 | <10 | <10 | <100 | <100 | <10 | <10 | - | - | - | + |
| Formulation Example 20 | <10$^4$ | <10$^4$ | <10$^4$ | <10$^4$ | <1000 | <1000 | <100 | <100 | | | + | ++ |

-: Normal state with no occurrence of off-odor, hyphae, or spores; +: Mold growth observed on a portion of the sample surface; ++: Occurrence of off-odor and mold growth on the sample surface.

As a result, as shown in Table 19, when the caprylhydroxamic acid was included in an amount of 1 to 15 wt% based on the total weight of the preservative (Formulation Examples 14 to 18), the number of viable bacteria, yeast, and molds was all significantly reduced. In contrast, Formulation Example 19, which contained 0.1 wt% of caprylhydroxamic acid based on the total weight of the preservative, showed an insignificant reduction in the number of bacteria and yeasts at week 1 and week 2, and mold growth was observed on a portion of the sample surface at week 4. In addition, Formulation Example 20 containing 20 wt% of caprylhydroxamic acid based on the total weight of the preservative showed an insignificant effect of reducing the number of bacteria and yeasts in all of week 1 to week 4, and at week 3, a mold growth was observed on a portion of the sample surfaces, and at week 4, off-odor occurred and mold growth was observed on the sample surfaces.

As a result, it was confirmed that a preservative containing caprylhydroxamic acid in a specific amount can exhibit excellent preservative efficacy.

**Experimental Example 9: Evaluation of Antiseptic Power Depending on Amount of Raspberry Ketone**

In this experimental example, the antiseptic power was evaluated according to an amount of raspberry ketone.

To this end, preservatives were prepared by varying the weight ratio in the composition shown in Table 20 below. The preservative of each formulation example was prepared by maintaining the weight ratio of hinokitiol:solvent and raspberry ketone at 1:19 while varying an amount of raspberry ketone.

Then, phase A to phase D were prepared with the components and amounts of Table 21, and mixed with 1 wt% of a preservative (Formulation Examples 21 to 27) to prepare a composition for external skin preparation.

[Table 20]

|  | Raw Material | Weight Ratio (Hinokitiol : Solvent : Raspberry Ketone) |
|---|---|---|
| Formulation Example 21 | Hinokitiol; 1,3-Propanediol; and Raspberry Ketone | 5 : 94 : 1 |
| Formulation Example 22 | Hinokitiol; 1,3-Propanediol; and Raspberry Ketone | 5: 92 : 3 |
| Formulation Example 23 | Hinokitiol; 1,3-Propanediol; and Raspberry Ketone | 5: 90 : 5 |
| Formulation Example 24 | Hinokitiol; 1,3-Propanediol; and Raspberry Ketone | 5: 85 : 10 |
| Formulation Example 25 | Hinokitiol; 1,3-Propanediol; and Raspberry Ketone | 5: 80 : 15 |
| Formulation Example 26 | Hinokitiol; 1,3-Propanediol; and Raspberry Ketone | 5 : 94.9 : 0.1 |
| Formulation Example 27 | Hinokitiol; 1,3-Propanediol; and Raspberry Ketone | 5 : 75 : 20 |

[Table 21]

|  | Ingredient | Amount (w/w) |
|---|---|---|
| Phase A | Distilled Water | Up to 100 |
|  | Glycerin | 3 |
|  | Sodium Chlorate | 0.9 |
|  | Polysorbate 20 | 0.9 |
| Phase B | Methyltrimethicone | 23 |
|  | Dimethicone | 4.5 |
|  | Propylene Glycol Dicaprate | 2 |
|  | PEG-10 Dimethicone | 3 |
| Phase C | Titanium Dioxide | 10.5 |
|  | Polyglyceryl-2 Triisostearate | 2.5 |
| Phase D | Dimethicone | 8.7 |
|  | Silica | 4.5 |

Thereafter, in order to evaluate the antiseptic power of the composition for external skin preparation, in the case of bacteria, *Escherichia coli, Pseudomonas aeruginosa,* and *Staphylococcus aureus* were mixed at the same concentration in 1:1:1 and seeded so that the initial concentration of bacteria per sample was about 10^6 CFU/g. For yeast and molds, *Candida albicans* and *Aspergillus brasiliensis* were seeded to an initial concentration of about 10^5 CFU/g, respectively. Thereafter, while the sample was stored at room temperature (25°C) for 4 weeks, 1 g of each sample was taken at 7-day intervals to check the number of remaining probiotics. In particular, in the case of molds, the presence or absence of off-odor and the presence or absence of hyphae and spores on the sample surface were checked for the sample.

[Table 22]

| CFU/g | Bacteria | | | | Yeast | | | | Mold | | | |
|---|---|---|---|---|---|---|---|---|---|---|---|---|
|  | Week 1 | Week 2 | Week 3 | Week 4 | Week 1 | Week 2 | Week 3 | Week 4 | Week 1 | Week 2 | Week 3 | Week 4 |
| Formulation Example 21 | <100 | <100 | <10 | <10 | <10 | <10 | <10 | <10 | - | - | - | + |
| Formulation Example 22 | <100 | <10 | <10 | <10 | <10 | <10 | <10 | <10 | - | - | - | + |
| Formulation Example 23 | <100 | <10 | <10 | <10 | <10 | <10 | <10 | <10 | - | - | - | + |
| Formulation Example 24 | <10 | <10 | <10 | <10 | <10 | <10 | <10 | <10 | - | - | - | - |

(continued)

| CFU/g | Bacteria | | | | Yeast | | | | Mold | | | |
|---|---|---|---|---|---|---|---|---|---|---|---|---|
| | Week 1 | Week 2 | Week 3 | Week 4 | Week 1 | Week 2 | Week 3 | Week 4 | Week 1 | Week 2 | Week 3 | Week 4 |
| Formulation Example 25 | <10 | <10 | <10 | <10 | <10 | <10 | <10 | <10 | - | - | - | - |
| Formulation Example 26 | <1000 | <100 | <10 | <10 | <100 | <100 | <10 | <10 | - | - | - | + |
| Formulation Example 27 | $<10^4$ | $<10^4$ | $<10^4$ | $<10^4$ | <1000 | <1000 | <100 | <100 | | | + | ++ |
| -: Normal state with no occurrence of off-odor, hyphae, or spores; +: Mold growth observed on a portion of the sample surface; ++: Occurrence of off-odor and mold growth on the sample surface. | | | | | | | | | | | | |

As a result, as shown in Table 22, when the amount of raspberry ketone was 1 to 15 wt% based on the total weight of the preservative (Formulation Examples 21 to 25), the number of bacteria, yeast, and molds was significantly reduced. In contrast, Formulation Example 26 containing 0.1 wt% of raspberry ketone based on the total weight of the preservative showed insignificant reduction of the number of bacteria and yeasts at week 1 and week 2, and mold growth was observed on a portion of the sample surface at week 4. In addition, Formulation Example 27 containing 20 wt% of raspberry ketone based on the total weight of the preservative showed an insignificant effect of reducing the number of bacteria and yeasts at week 1 to at week 4, and mold growth was observed on a portion of the sample surface at week 3, and off-odor occurred and mold growth was observed on the sample surfaces at week 4.

As a result, it was confirmed that a preservative containing raspberry ketone in a specific amount can exhibit excellent preservative efficacy.

The description of the present disclosure described above is for illustration, and those of ordinary skill in the art to which the present disclosure pertains will understand that it can be easily modified into other specific forms without changing the technical spirit or essential features of the present disclosure. Therefore, it should be understood that the embodiments described above are exemplary and not restrictive in all aspects.

## Claims

1. A preservative for external skin preparation, comprising: hinokitiol and at least one solvent selected from the group consisting of alkanediol having 3 to 10 carbon atoms, alkanetriol having 3 to 6 carbon atoms, dipropylene glycol, glyceryl caprylate, ethylhexylglycerin, and phenoxyethanol.

2. The preservative for external skin preparation of claim 1, wherein hinokitiol and the solvent are included in a weight ratio of 1:19 to 1:1000.

3. The preservative for external skin preparation of claim 1, wherein alkanediol having 3 to 10 carbon atoms is at least one selected from the group consisting of 1,3-propanediol, methylpropanediol, 2,3-butanediol, 1,3-butanediol, 1,2-pentanediol, 1,5-pentanediol, 1,2-hexanediol, 1,2-heptanediol, 1,2-octanediol, and 1,2-decanediol.

4. The preservative for external skin preparation of claim 1, wherein the alkanetriol having 3 to 6 carbon atoms is glycerin, 1,2,6-hexanetriol, or a combination thereof.

5. The preservative for external skin preparation of claim 1, wherein the solvent is at least one selected from the group consisting of 1,3-propanediol, methylpropanediol, 2,3-butanediol, 1,3-butanediol, 1,2-pentanediol, 1,5-pentanediol, 1,2-hexanediol, 1,2-heptanediol, 1,2-octanediol, 1,2-decanediol, glycerin, 1,2,6-hexanetriol, dipropylene glycol, glyceryl caprylate, ethylhexylglycerin, and phenoxyethanol.

6. The preservative for external skin preparation of claim 1, wherein the solvent is 1,2-pentanediol, 1,5-pentanediol, 1,2-octanediol, glyceryl caprylate, or a combination thereof.

7. The preservative for external skin preparation of claim 1, further comprising caprylhydroxamic acid or raspberry ketone.

8. The preservative for external skin preparation of claim 7, wherein the weight ratio of hinokitiol to the solvent and either caprylhydroxamic acid or raspberry ketone is 1:19 to 1:1000.

9. The preservative for external skin preparation of claim 7, wherein an amount of the caprylhydroxamic acid or raspberry ketone is 1 to 20 wt% based on the total weight of the preservative.

10. A cosmetic composition comprising the preservative for external skin preparation of any one of claims 1 to 9.

11. The cosmetic composition of claim 10, wherein the preservative for external skin preparation is included in an amount of 0.0001 to 50 wt% based on the total weight of the cosmetic composition.

12. A pharmaceutical composition for external skin preparation, the pharmaceutical composition comprising the preservative for external skin preparation of any one of claims 1 to 9.

13. The pharmaceutical composition of claim 12, wherein the preservative for external skin preparation is included in an amount of 0.0001 to 50 wt% based on the total weight of the pharmaceutical composition.

## INTERNATIONAL SEARCH REPORT

| International application No. |
|---|
| **PCT/KR2024/013163** |

| A. | CLASSIFICATION OF SUBJECT MATTER |
|---|---|

**A61K 8/35**(2006.01)i; **A61K 8/34**(2006.01)i; **A61K 8/37**(2006.01)i; **A61Q 19/00**(2006.01)i; **A61K 47/10**(2006.01)i; **A61K 47/14**(2006.01)i; **A61Q 17/00**(2006.01)i

According to International Patent Classification (IPC) or to both national classification and IPC

| B. | FIELDS SEARCHED |
|---|---|

Minimum documentation searched (classification system followed by classification symbols)

A61K 8/35(2006.01); A61K 31/075(2006.01); A61K 36/42(2006.01); A61K 8/33(2006.01); A61K 8/9717(2017.01); A61K 8/9761(2017.01); A61P 17/00(2006.01); C07C 69/28(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 히노키티올(Hinokitiol), 알칸디올(Alkanediol), 알칸트리올(Alkanetriol), 피부 외용제(skin external application)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | KR 10-2062006 B1 (L&P COSMETIC CO., LTD.) 03 January 2020 (2020-01-03) See claims 1 and 5; and paragraphs [0010]-[0011], [0044]-[0046], [0052]-[0053] and [0067]-[0068]. | 1-5,10-13 |
| Y | | 6-9 |
| Y | KR 10-1699468 B1 (PHYTOCHEM TECH CO., LTD.) 25 January 2017 (2017-01-25) See claim 4; and paragraphs [0012] and [0021]. | 6-9 |
| A | JP 3881411 B2 (KOSE CORP. et al.) 14 February 2007 (2007-02-14) See entire document. | 1-13 |
| A | JP 2002-047123 A (ASAHI KASEI CORP.) 12 February 2002 (2002-02-12) See entire document. | 1-13 |

☑ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **06 December 2024** | **06 December 2024** |

| Name and mailing address of the ISA/KR | Authorized officer |
|---|---|
| **Korean Intellectual Property Office Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**EP 4 781 972 A1**

**INTERNATIONAL SEARCH REPORT**

| International application No. |
| --- |
| **PCT/KR2024/013163** |

**C.    DOCUMENTS CONSIDERED TO BE RELEVANT**

| Category* | Citation of document, with indication, where appropriate, of the relevant passages | Relevant to claim No. |
| --- | --- | --- |
| A | JP 2005-206573 A (NIPPON FINE CHEMICAL CO., LTD.) 04 August 2005 (2005-08-04)<br>See entire document. | 1-13 |

Form PCT/ISA/210 (second sheet) (July 2022)

31

**INTERNATIONAL SEARCH REPORT**
Information on patent family members

International application No.

**PCT/KR2024/013163**

| Patent document cited in search report | | | Publication date (day/month/year) | Patent family member(s) | | | Publication date (day/month/year) |
|---|---|---|---|---|---|---|---|
| KR | 10-2062006 | B1 | 03 January 2020 | None | | | |
| KR | 10-1699468 | B1 | 25 January 2017 | KR 10-2016-0117668 | | A | 11 October 2016 |
| JP | 3881411 | B2 | 14 February 2007 | JP | 10-114670 | A | 06 May 1998 |
| JP | 2002-047123 | A | 12 February 2002 | None | | | |
| JP | 2005-206573 | A | 04 August 2005 | JP | 4589050 | B2 | 01 December 2010 |

Form PCT/ISA/210 (patent family annex) (July 2022)

**EP 4 781 972 A1**

**Patent documents cited in the description**

- KR 1020230124816
- KR 1020240036722